(19) Europäisches Patentamt
European Patent Office
Office européen des brevets

(11) Veröffentlichungsnummer: **0 257 632**
**B1**

(12) # EUROPÄISCHE PATENTSCHRIFT

(45) Veröffentlichungstag der Patentschrift: **12.12.90**

(51) Int. Cl.[5]: **C08F 226/00,** C08F 220/28, C08F 216/14, C12N 11/08

(21) Anmeldenummer: **87112388.1**

(22) Anmeldetag: **26.08.87**

(54) **Vernetzte Polymerisate mit Carbonatestergruppen und Verfahren zu ihrer Herstellung.**

(30) Priorität: **28.08.86 DE 3629176**

(43) Veröffentlichungstag der Anmeldung:
**02.03.88 Patentblatt 88/9**

(45) Bekanntmachung des Hinweises auf die Patenterteilung:
**12.12.90 Patentblatt 90/50**

(84) Benannte Vertragsstaaten:
**AT BE CH DE FR GB IT LI NL SE**

(56) Entgegenhaltungen:
**EP-A- 0 161 464**
**US-A- 2 967 173**
**US-A- 3 012 010**

(73) Patentinhaber: **HOECHST AKTIENGESELLSCHAFT, Postfach 80 03 20, D-6230 Frankfurt am Main 80(DE)**

(72) Erfinder: **Mauz, Otto, Dr., Heidestr. 21, D-6237 Liederbach(DE)**
Erfinder: **Noetzel, Siegfried, Dr., An den Römergärten 3, D-6233 Kelkheim (Taunus)(DE)**
Erfinder: **Sauber, Klaus, Dr., Friedrich Ebert-Str. 15, D-6231 Schwalbach am Taunus(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung bezieht sich auf vernetzte Polymerisate, die überwiegend in Form von kugelförmigen porösen Teilchen vorliegen und auf Basis von Glycerincarbonatgruppen enthaltenden Monomeren, vernetzenden Monomeren und gegebenenfalls weiteren monoethylenisch ungesättigten Monomeren aufgebaut sind. Solche Polymerisate eignen sich sehr gut als Trägermaterialien für die Immobilisierung von biologisch aktiven Substanzen.

Es ist bekannt, biologisch aktive Substanzen, wie Enzyme, Antikörper, Antigene, Hormone und dergleichen unter Erhalt ihrer Aktivität an polymere Trägermaterialien über kovalente Bindungen zu fixieren, um auf diesem Weg beispielsweise Enzyme zu stabilisieren, zu reinigen oder wasserunlöslich zu machen. Solchermaßen immobilisierte biologisch aktive Substanzen bieten erhebliche Vorteile gegenüber der löslichen Form: Zum einen ist die Abtrennbarkeit durch Sedimentation nach Beendigung einer Reaktion vereinfacht, zum anderen ist die Stabilität und Wiederverwendbarkeit der Präparate und ein Vielfaches erhöht.

Es ist auch bekannt, Polymere und Copolymere des Vinylencarbonats zur Bindung von biologisch aktiven Substanzen zu verwenden (vgl. DE-A 2 407 340, DE-A 2 552 510). Die nach Fixierung der biologisch aktiven Substanzen noch vorhandenen Cyclocarbonatgruppen werden nach einer Variante der darin beschriebenen Verfahren in Hydroxygruppen überführt.

Dieses bekannte Polymerträgermaterial auf Basis von Vinylencarbonat als reaktive Monomereinheit hat zwar gegenüber anderen Trägermaterialien den Vorteil geringer unspezifischer Adsorption biologisch aktiver Substanzen, ihm fehlt jedoch die perlförmige Morphologie und die erforderliche Porosität. Dadurch ist es beispielsweise für Säulenverfahren nicht anwendbar.

Unregelmäßig geformte Copolymerisate des Vinylencarbonats fallen bei der Polymerisation in einem organischen unpolaren Dispergiermittel in Gegenwart von bestimmten nichtionogenen Dispersionsstabilisatoren an (vgl. DE-A 25 56 759). Auch diese Polymerisate besitzen keine Perlform und auch nicht das notwendige Maß an Porosität.

Weiterhin sind Vinylencarbonat-Perlpolymerisate bekannt, die zur Herstellung von trägergebundenen, biologisch aktiven Substanzen verwendet werden können (vgl. DE-A 3 243 591). Die Herstellung dieser Trägercopolymerisate muß aber in Kohlenwasserstoffen bzw. Paraffinöl in Anwesenheit bestimmter Dispersionsstabilisatoren erfolgen.

Auch sind modifizierte Polymerisate auf Basis von Polyvinylencarbonat und/oder Polyhydroxymethylen bekannt, wobei die Modifizierung durch bestimmte, in das Polymerisat eingebaute alkoxylierte Verbindungen erfolgt (vgl. EP-A 161 464). Diese Polymerisate, bei deren Herstellung bestimmte Dispersionsstabilisatoren eingesetzt werden müssen, eignen sich ebenfalls als Trägermaterialien für biologisch aktive Substanzen oder für die Affinitätschromatographie. Auch hier ist in der Regel die aufwendige inverse Suspensionspolymerisation erforderlich.

Es bestand somit die Aufgabe, Polymerisate zur Immobilisierung von biologisch aktiven Substanzen wie z.B. Enzymen zu finden, welche sich auf eine sehr einfache Weise herstellen lassen und eine sehr gute Bindefähigkeit gegenüber biologisch aktiven Wirkstoffen aufweisen.

Dies wurde dadurch erreicht, daß poröse, vernetzte Glycerincarbonatestergruppen enthaltende Polymerisate in Perl- oder Granulatform eingesetzt werden.

Die Erfindung betrifft somit ein vernetztes Polymerisat, bestehend im wesentlichen aus A) 1 bis 99 Gew.-% Einheiten, die sich von Methacrylsäure-glycerincarbonat-ester, Acrylsäure-glycerincarbonat-ester, Glycerincarbonat-vinylether und/oder Glycerincarbonat-allylether ableiten, B) 99 bis 1 Gew.-% Einheiten, die sich von N,N'-Divinylethylenharnstoff und/oder N,N'-Divinylpropylenharnstoff ableiten, wobei die Summe der Einheiten stets 100 Gew.-% ist und wobei die Polymerisatteilchen im wesentlichen kugelförmige Gestalt, eine mittlere Teilchengröße von 10 bis 600 µm und einen mittleren Porendurchmesser von 5 bis 1000 nm aufweisen.

Die Erfindung betrifft auch ein Verfahren zur Herstellung des genannten Polymerisats durch Copolymerisation der Monomeren in einem flüssigen Dispersionsmittel, welches unter den Polymerisationsbedingungen die Monomeren und das Polymerisat nicht löst, in Gegenwart eines radikalisch wirksamen Initiators und weiterer Hilfsstoffe sowie eines Stoffes, welcher sich in den Monomeren gut löst oder mit ihnen mischbar ist und im Dispersionsmittel praktisch unlöslich ist (Inertmittel), dadurch gekennzeichnet, daß A') 1 bis 99 Gew.-%, bezogen auf das Monomerengemisch, Methacrylsäure-glycerincarbonat-ester, Acrylsäure-glycerincarbonat-ester, Glycerincarbonat-vinylether und/oder Glycerincarbonat-allylether und B') 99 bis 1 Gew.-%, bezogen auf das Monomerengemisch, N,N'-Divinylethylenharnstoff und/oder N,N'-Divinylpropylenharnstoff, wobei die Summe der Monomeren stets 100 Gew.-% ist, in Gegenwart von 50 bis 300 Gew.-%, bezogen auf die Summe der Monomeren, Inertmittel copolymerisiert werden.

Eine bevorzugte Ausführungsform des Verfahrens enthält als zusätzliche Komponente C') 0,1 bis 10 Gew.-%, bezogen auf das Monomerengemisch, Vinylacetat, Methylmethacrylat, Butylacrylat und/oder Styrol.

Die Erfindung betrifft schließlich auch die Verwendung der so erhaltenen Polymerisate als Trägermaterialien zur Herstellung von trägergebundenen, biologisch aktiven Substanzen.

Das erfindungsgemäße Polymerisat besteht zu A) 1 bis 99 Gew.-%, vorzugsweise 3 bis 60 Gew.-%,

insbesondere 8 bis 50 Gew.-% aus Einheiten die sich von einem glycerincarbonatgruppenhaltigen Monomer A') ableiten, B) 99 bis 1 Gew.-%, vorzugsweise 97 bis 40 Gew.-%, insbesondere 92 bis 50 Gew.-% aus Einheiten, die sich von einem vernetzenden Monomer B') ableiten, und gegebenenfalls C) 0,1 bis 10 Gew.-%, vorzugsweise 0,1 bis 5 Gew.-% aus Einheiten, die sich von einem monoethylenisch ungesättigten nicht hydrophilen und nichtvernetzenden Monomer C') ableiten. Die Gewichtsprozente sind jeweils auf das Gesamtpolymerisat bezogen.

Geeignete glycerincarbonatgruppenhaltige Monomere A') sind beispielsweise Acrylsäure-glycerin-carbonat-ester, Glycerincarbonat-vinylether und/oder Glycerincarbonat-allylether, vorzugsweise Methacrylsäure-glycerin-carbonat-ester, allein oder im Gemisch. Die Herstellung der glycerincarbonatgruppenhaltigen Monomeren erfolgt beispielsweise nach den in den deutschen Patentanmeldungen P 35 29 263.6 und P 36 00 602.5 (eingereicht am 16.08.85 bzw. am 11.01.86) beschriebenen Verfahren durch Einleiten von Kohlendioxyd in die in einem organischen Lösungsmittel gelösten entsprechenden epoxydgruppenhaltigen Monomeren in Gegenwart von geeigneten Katalysatoren, wobei der Epoxydring durch Einbau des Kohlendioxydmoleküls in den Carbonatring übergeführt wird.

Geeignete vernetzende Monomere B') sind z.B. N,N'-Divinylpropylenharnstoff, bevorzugt jedoch N,N'-Divinylethylenharnstoff, allein oder im Gemisch.

Geeignete monoethylenisch ungesättigte, nicht hydrophile und nichtvernetzende Monomere C') sind Vinylacylate, Alkylacrylate, Alkylmethacrylate, Styrol, Styrolderivate, vorzugsweise Vinylacetat, Methylmethacrylat, Butylacrylat und Styrol, allein oder im Gemisch.

Im erfindungsgemäßen Verfahren zur Herstellung des erfindungsgemäßen Polymerisats werden die Monomeren in Gegenwart eines radikalisch wirksamen Initiators und weiterer Hilfsstoffe im Suspensions-, Lösungs- oder Fällungspolymerisationsverfahren polymerisiert. Bevorzugt ist die Suspensionspolymerisation in Wasser als Suspensionsmittel bei einer Temperatur von 20 bis 120 C, vorzugsweise von 25 bis 90 °C.

Als radikalisch wirksame Initiatoren kommen solche in Betracht, die in der Monomerphase gut und in Wasser schwer löslich sind. Beispiele hierfür sind organische Peroxide, wie Di-tert.-butylperoxid, Dibenzoylperoxid, Bis(o-methylbenzoyl)peroxid, tert.-Butylhydroperoxyd, Cumolhydroperoxid, Di-isopropylperoxidicarbonat, Cyclohexanonperoxid oder aliphatisc he Azoverbindungen, wie α,α'-Azodiisobuttersäurenitril, Azobis-cyanvaleriansäure, 1,1'-Azo-cyclo-hexan-1,1'-dicarbonsäurenitril und Azodicarbonamid.

Bei der Suspensionspolymerisation werden Stabilisatoren und/oder Dispergierhilfsmittel verwendet wie beispielsweise Polyvinylpyrrolidon, Polyacrylamid, Polyvinylalkohol, Hydroxyethylcellulose.

Um eine möglichst hohe Porosität der Perlpolymerisate zu erreichen, werden dem Polymerisationssystem oder vorzugsweise den Monomeren bestimmte inerte, flüssige Komponenten (Inertmittel) zugesetzt. Hierunter sollen solche Stoffe verstanden sein, in denen sich die Monomeren gut lösen oder mit ihnen mischbar, andererseits aber im Dispergiermittel praktisch unlöslich und damit mit diesem nicht mischbar sind. Nach ihrem Verhalten zu den entsprechenden Copolymeren kann man die Inertmittel in Quellungs- und/oder Fällungsmittel einteilen. Die Inertmittel nehmen an der Polymerisation nicht teil, werden jedoch vom Polymerisat umhüllt und bei der Aufarbeitung wieder herausgelöst. Dadurch entstehen permanente Poren. Die Porengröße ist durch Art und Menge des Inertmittels beeinflußbar, hängt aber auch von der Menge an vernetzender Komponente ab.

Die bei der Polymerisation verwendeten Inertmittel, in denen die Monomeren gelöst werden, dürfen im vorliegenden Fall mit den ethylenischen Doppelbindungen und den Glycerincarbonatgruppen der Monomeren nicht reagieren.

Bevorzugte Inertmittel sind Pentanol, Heptylalkohol, 2-Ethylhexanol, Nonylalkohol, Decylalkohol, Laurylalkohol, Cyclohexanol und Oxoalkohole z.B. TCD-Alkohol M

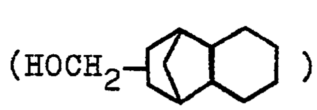

Die Inertmittel werden in einer Menge von 50 bis 300 Gew.-%, vorzugsweise 100 bis 250 Gew.-%, insbesondere 125 bis 200 Gew.-%, bezogen auf die Gesamtmenge der eingesetzten Monomeren, verwendet. Sie können allein oder im Gemisch eingesetzt werden.

Das erfindungsgemäße Verfahren wird zweckmäßigerweise in einem mit einer Rührvorrichtung versehenen Reaktionsgefäß durchgeführt. Die Teilchengröße des Perlpolymerisates wird in bekannter Weise durch die Rührgeschwindigkeit und das Phasenverhältnis eingestellt. Besonders vorteilhaft ist die Verwendung eines senkrecht stehenden, zylindrischen Gefäßes mit flachem Boden, das mit einem koaxial angebrachten Rührer versehen ist, dessen Welle bis fast auf den Gefäßboden reicht. Das Reaktionsgefäß ist vorzugsweise vakuumfest und kann mit Rückflußkühler, Zulauftrichter, Gaseinleitungsrohr und Temperaturmeßgerät versehen werden. Die Beheizung und Kühlung des Gefäßes erfolgt im allgemeinen durch ein Flüssigkeitsbad, z.B. ein Ölbad oder Wasserbad.

Es ist vorteilhaft, das erfindungsgemäße Verfahren unter Ausschluß von Luftsauerstoff durchzuführen. Das Reaktionsgefäß wird daher vor Beginn mit einem inerten Gas, vorzugsweise Stickstoff, gespült.

Nach Beendigung der Polymerisationsreaktion werden die nicht umgesetzten Monomeren aus dem Reaktionsgefäß entfernt z.B. durch Verdampfen bei vermindertem Druck, vorzugsweise einem Druck von 0,1 bis 15 Torr. Nach der Entfernung der Restmonomeren wird das Dispersionsmittel vom festen Polymeren abgetrennt, z.B. durch Dekantieren, Filtrieren oder Absaugen des Überstandes. Anschließend wird das Polymerisat, falls erforderlich, mit leichtsiedenden organsichen Lösungsmitteln, z.B. einem Kohlenwasserstoff, einem niederen Alkohol oder Aceton gewaschen und schließlich getrocknet. Die Trocknung des Polymerisates erfolgt bei einer Temperatur von zumeist 20 bis 100 °C, vorzugsweise von 40 bis 80 °C; eine Trocknung unter vermindertem Druck ist dabei empfehlenswert.

Das erfindungsgemäße Perlpolymerisat besteht aus überwiegend kugelförmigen Teilchen, deren mittlere Teilchengröße im trocknen, ungequollenen Zustand 10 bis 600 µm, vorzugsweise 20 bis 400 µm beträgt und die vorzugsweise eine enge Teilchengrößenverteilung aufweisen. Das jeweilige Optimum der Teilchengröße hängt dabei vor allem von dem speziellen Einsatzgebiet ab. Bei einem ohne Druck durchgeführten Säulenverfahren wird man beispielsweise die Teilchengröße innerhalb der vorstehend genannten Grenzen entsprechend größer wählen können als bei einem Druckverfahren. Die Perlen des erfindungsgemäßen Perlpolymerisates sind überwiegend als makroporöse Perlen ausgebildet. Dies drückt sich in dem sich daraus ergebenden erfindungsgemäßen mittleren Porendurchmesser im Bereich von 5 bis 1000 nm, vorzugsweise 10 bis 800 nm aus.

Die Bestimmung des Porendurchmessers (Porenvolumens) erfolgt in der Weise, daß zunächst das Porenvolumen gemäß der Kapillardruckmethode (Quecksilberporosimetrie) bestimmt wird. Daneben ist eine Porengrößenbestimmung auch durch Rasterelektronenmikroskopie möglich.

Die erfindungsgemäßen Polymerisate eignen sich für die Immobilisierung von biologisch aktiven Substanzen durch Ausbildung einer kovalenten Bindung. Sie eignen sich aber auch, gegebenenfalls nach Inaktivierung der Glycerincarbonatgruppen, für andere Zwecke wie z.B. die Affinitätschromatographie usw.

Unter dem Begiff "biologisch aktive Substanzen" seien die bekannten in vivo oder in vitro wirksamen natürlichen oder künstlich hergestellten Stoffe verstanden, beispielsweise Enzyme, Aktivatoren, Inhibitoren, Antigene, Antikörper, Vitamine, Hormone, Effektoren, Antibiotika, Proteine. Der Begriff Proteine umfaßt dabei auch Proteine mit bestimmten Nicht-Proteinsubstituenten wie Metallionen, Polysacchariden, Porphyringruppen, Adenindinucleotid, Ribonucleinsäure, Phospholipide etc. Auch Polypeptidfragmente, beispielsweise die aktiven Teile von Enzymmolekülen, fallen unter den Begriff "biologisch aktive Substanzen".

Von den vorstehend genannten biologisch aktiven Substanzen sind die Enzyme bevorzugt. Beispiele für Enzyme sind Urease, Penicillinacylase,D-Aminosäureoxidase, Adenyldesaminase, Alkohol-Dehydrogenase, Asparaginase, Carboxypeptidase, Chymotrypsin, Diphosphoesterase, α-Glucosidase, Glucose-Isomerase, Glucose-Oxidase, Glucose-6-phosphat-Dehydrogenase, Hexokinase, Invertase, β-Lactamase, Lactase, Lactat-Dehydrogenase, versch. Lectine, NAD-Kinase, Neuraminidase, Papain, Peroxidase, Phosphatasen (alkalisch und sauer), 5'-Phosphodiesterase, Pyruvat Kinase, Ribonuclease, Trypsin.

Beispiele für andere biologisch aktive Substanzen sind Hormone, wie Insulin und die verschiedensten Hypophysen-Hormone, Proteine der gamma-Globulinfraktion, z.B. Antihämophiliefaktor, die Blutgerinnungsfaktoren, spezielle Antikörper, z.B. Hepatitis-, Poliomyelitis-, Finnen-, Mumps-, Influenza- oder Kaninchenantikörper, Antigene, wie Hepatitis-, Polymomyelitis-, Finnen-, Mumps-, Infuenza- oder Kaninchenantigene zur Reinigung oder Stimulierung geeigneter Antikörperreaktionen, wobei das Antigen (nach dem Unlöslichmachen) in der unlöslichen Form verbleibt und folglich nicht in den Körper eindringen und diesen schädigen kann, sowie allgemeine Körperproteine, wie Hämoglobin oder Albumin.

Die Bindung der biologisch aktiven Substanzen an das polymere Trägermaterial ist an sich bekannt und erfolgt allgemein so, daß das trockene Trägermaterial beispielsweise zu einer Enzymlösung gegeben wird, die mittels einer Pufferlösung, z.B. 1,5-molarer Kaliumphosphatlösung in Wasser, auf einem bestimmten pH-Wert eingestellt wird. Nach einer Fixierzeit, die 1 bis 72 Stunden betragen kann, wird bei einer bestimmten Temperatur (z.B. 23 °C) das Trägermaterial mit 1-molarer Kochsalzlösung und mit der Pufferlösung gut gewaschen. Am feuchten Trägermaterial wird dann nach Zusatz des zu spaltenden Substrates die spezifische Aktivität, bestimmt, beispielsweise durch automatische Titration.

Die erfindungsgemäßen neuen Polymerisate zeigen folgende Vorteile:

Sie lassen sich aus billigen, handelsüblichen Ausgangsprodukten herstellen; bei der Suspensionpolymerisation kann mit Wasser als Suspensionsmittel gearbeitet werden; Kohlenwasserstoffe und Chlorkohlenwasserstoffe, wie sie beider inversen Suspensionspolymerisation erforderlich sind, werden vermieden.

Die perlförmigen Poylmerisate haben eine sehr gute Bindefähigkeit für biologisch aktive Substanzen.

Beispiele

1) bis 6) In einem mit Rückflußkühler, Rührer, Thermometer und Stickstoff-Einleitungsrohr versehenen Rundkolben wurden 200 ml entmineralisiertes Wasser, 3,2 g Dinatriumhydrogenphosphat und 2,0 g Polyvinylpyrrolidon vom Molgewicht 360 000 vorgelegt und das Gemisch ca. 20 Minuten bei 25 °C bis zur vollständigen Lösung des Polyvinylpyrrolidons verrührt. Dann erfolgte die Zugabe einer Lösung, die aus den Komponenten A'), B') und gegebenenfalls C') sowie Inertmittel und 2 g Azoisobutyronitril bestand. Das Gemisch wurde dann unter Rühren und Stickstoffüberlagerung langsam auf eine Temperatur von 65 °C erhitzt und 7 Stunden bei dieser Temperatur mittels eines thermostatisierten Ölbades gehalten. Nach dem Abkühlen des Ansatzes auf ca. 25 °C wurde das erhaltene Perlpolymerisat über eine Nutsche abgesaugt, dreimal mit jeweils 1 Liter Wasser 30 Minuten lang verrührt und abgesaugt, viermal mit jeweils 1 Liter Methanol 30 Minuten lang verrührt und abgesaugt und zweimal mit jeweils 1 Liter Aceton 30 Minuten lang verrührt und abgesaugt. Das acetonfeuchte Perlpolymerisat wurde gesiebt und im Trockenschrank bei 50 °C und 0,267 bar über Nacht unter Stickstoff getrocknet.

Die Ausbeuten, Korngrößenverteilung gemäß Siebanalyse und gegebenenfalls die mittleren Porendurchmesser sowie die zu deren Bestimmung erforderlichen Porenvolumina sind in der Tabelle 1 angeführt.

Tabelle 1

| | Beispiele 1 | 2 | 3 | 4 | 5 | 6 |
|---|---|---|---|---|---|---|
| Komp. A'. [g] | | | | | | |
| Methacrylsäure-glycerincarbonatester | 50 | 20 | 40 | 40 | - | - |
| Allylglycerincarbonatether | - | - | - | - | - | 20 |
| Vinylglycerincarbonatether | - | - | - | - | 50 | - |
| Komp. B': [g] | | | | | | |
| N,N'-Divinylethylenharnstoff | 50 | 80 | 50 | 50 | 50 | 80 |
| Komp. C': [g] | | | | | | |
| Vinylacetat | - | - | - | 10 | - | - |
| Methylmethacrylat | - | 10 | - | - | - | - |
| Inertmittel: [g] | | | | | | |
| Cyclohexanol | 108 | 108 | 108 | 108 | 108 | 108 |
| Laurylalkohol | 12 | 12 | 12 | 12 | 12 | 12 |
| Ausbeute [g = % d.Th.] | 75 | 86 | 90 | 90,6 | 84,5 | 92,0 |
| Korngröße: > 300 µm [%] | - | - | - | - | - | - |
| 200-300 µm | - | - | 3,3 | - | - | - |
| 100-200 µm | 36,0 | 53,0 | 26,9 | 35,4 | 53,4 | 51,7 |
| 50-100 µm | 60,2 | 46,3 | 68,1 | 60,0 | 42,8 | 43,8 |
| < 50 µm | 3,8 | 0,7 | 1,7 | 4,5 | 3,8 | 4,5 |
| spez. Porenvolumen [$cm^3$/g] | 0,92 | - | - | 0,68 | - | 0,74 |
| Porendurchmesser [nm] | 60 | - | - | 75 | - | 54 |

7) bis 11) Zu 0,2 g eines nach einem der Beispiele 1 bis 5 hergestellten Trägermaterials wurde die Lösung einer biologisch aktiven Substanz in einer Pufferlösung gegeben. Nach dem Fixieren während 72 Stunden bei 23 °C (Beispiel 10 Fixierzeit 16 Stunden) wurden die Perlen mit 1-molarer Kochsalzlösung und mit Pufferlösung gründlich gewaschen. Die Ausbeute an nutschenfeuchtem Material, gemessen mittels Titrierautomat auf einem Substrat, das entsprechende Trockengewicht sowie die nach Bilanzierung von Ausgangs-und Waschwasseraktivität ermittelte Fixierausbeute (= Verhältnis Aktivität auf Träger: angebotener Aktivität) und der η-Wert (η= gefundene Aktivität / angebotene Aktivität minus Waschwasser-Aktivität) sind in Tabelle 2 angeführt. Die Aktivität (U) ist die Umwandlung von 1 µmol Substanz pro Minute, die spezifische Aktivität =

$$\frac{\text{Umwandlung von 1 µmol Substanz}}{\text{Minute x Gramm}}$$

**Tabelle 2**

| | Beispiele 7 | 8 | 9 | 10 | 11 |
|---|---|---|---|---|---|
| Trägermaterial gem. Beispiel | 1 | 2 | 3 | 4 | 5 |
| Biol. aktive Substanz (Lösung) [µl] | | | | | |
| Penicillin-Acylase | 1200 | 1200 | - | - | - |
| Trypsin | - | - | 1000 | - | - |
| Urease | - | - | - | 1000 | - |
| Chymotrypsin | - | - | - | - | 700 |
| enthaltend [µg/ml] | 30 | 30 | 6,25 | 30 | 6 |
| entspr. [U/ml] | 220 | 240 | 475 | 45 | 1001 |
| Pufferlösung [molar] | | | | | |
| Kaliumphosphat | 1,5 | 1,5 | 1,0 | 1,0 | 1,0 |
| Benzamidin | - | - | $1,6 \times 10^{-2}$ | - | - |
| pH-Wert | 7,6 | 7,6 | 7,8 | 8,0 | 8,0 |

Fortsetzung der Tabelle 2

| | Beispiele 7 | 8 | 9 | 10 | 11 |
|---|---|---|---|---|---|
| Titrierbedingungen: | | | | | |
| Temp. [°C] | 37 | 37 | 37 | 30 | 37 |
| pH-Wert | 7,8 | 7,8 | 8,1 | 6,1 | 8,9 |
| Substrat | A | A | B | C | D |
| Ausbeute [mg] (nutschenfeucht) | 456 | 544 | 495 | 497 | 566 |
| entspr. [U/g] | 296 | 288 | 356 | 55 | 510 |
| bez. auf Trockengewicht [U/g] | 675 | 785 | 880 | 137 | 1445 |
| Fixierausbeute [%] | 51 | 60 | 37 | 60 | 41 |
| η-Wert | 0,57 | 0,61 | 0,38 | 0,65 | 0,41 |

Substrat: A = penicillinsaures Kalium
B = N'-Benzoyl-L-argininethyl-esterhydrochlorid (BAEE)
C = Harnstoff
D = Acetyl-tyrosin-ethylester

**Patentansprüche für die Vertragsstaaten BE, CH, DE, FR, GB, IT, LI, NL, SE**

1. Vernetztes Polymerisat, bestehend im wesentlichen aus A) 1 bis 99 Gew.-% Einheiten, die sich von Methacrylsäure-glycerincarbonat-ester, Acrylsäure-glycerincarbonat-ester, Glycerincarbonat-vinylether und/oder Glycerincarbonat-allylether ableiten und B) 99 bis 1 Gew.-%, Einheiten, die sich von N,N'-Divinylethylenharnstoff und/oder N,N'-Divinylpropylenharnstoff ableiten, wobei die Summe der Einheiten stets 100 Gew.-% ist und wobei die Polymerisatteilchen eine im wesentlichen kugelförmige Gestalt, eine mittlere Teilchengröße von 10 bis 600 µm und einen mittleren Porendurchmesser von 5 bis 1000 nm aufweisen.

2. Polymerisat nach Anspruch 1, dadurch gekennzeichnet, daß Methacrylsäure-glycerincarbonat-ester eingesetzt wird.

3. Polymerisat nach Anspruch 1 oder 2, dadurch gekennzeichnet, daß es zusätzlich C) 0,1 bis 10 Gew.-%, bezogen auf das Gesamtpolymerisat, Einheiten enthält, welche sich von Vinylacetat, Methylmethacrylat, Butylacrylat und/oder Styrol ableiten.

4. Verfahren zur Herstellung eines vernetzten Polymerisats durch Copolymerisation von Monomeren in einem flüssigen Dispersionsmittel, welches unter den Polymerisationsbedingungen die Monomeren und das Polymerisat nicht löst, in Gegenwart eines radikalisch wirksamen Initiators und weiterer Hilfsstoffe sowie eines Stoffes, welcher sich in den Monomeren gut löst oder mit ihnen mi schbar ist und im Dispersionsmittel praktisch unlöslich ist (Inertmittel), dadurch gekennzeichnet, daß A') 1 bis 99 Gew.-% Methacrylsäure-glycerincarbonat-ester, Acrylsäure-glycerincarbonat-ester, Glycerincarbonat-vinylether und/oder Glycerincarbonat-allylether und B') 99 bis 1 Gew.-% N,N'-Divinylethylenharnstoff und/oder N,N'-Divinylpropylenharnstoff, wobei die Summe der Monomeren stets 100 Gew.-% ist, in Gegenwart von 50 bis 300 Gew.-% Inertmittel, bezogen auf die Summe der Monomeren, copolymerisiert werden.

5. Verfahren nach Anspruch 4, dadurch gekennzeichnet, daß zusätzlich als Komponente C') 0,1 bis 10 Gew.-%, bezogen auf das Monomerengemisch, Vinylacetat, Methylmethacrylat, Butylacrylat und/oder Styrol copolymerisiert werden.

6. Verfahren nach einem oder mehreren der Ansprüche 4 und 5, dadurch gekennzeichnet, daß die Copolymerisation als Suspensionspolymerisation in Wasser als Suspensionsmittel bei einer Temperatur von 20 bis 120 °C durchgeführt wird.

7. Verwendung der Polymerisate nach Anspruch 1 als Trägermaterialien für die Immobilisierung biologisch aktiver Substanzen.

8. Verwendung nach Anspruch 7, dadurch gekennzeichnet, daß die biologisch aktiven Substanzen Enzyme sind.

**Patentansprüche für den Vertragsstaat: AT**

1. Verfahren zur Herstellung eines vernetzten Polymerisats durch Copolymerisation von Monomeren in einem flüssigen Dispersionsmittel, welches unter den Polymerisationsbedingungen die Monomeren und das Polymerisat nicht löst, in Gegenwart eines radikalisch wirksamen Initiators und weiterer Hilfsstoffe sowie eines Stoffes, welcher sich in den Monomeren gut löst oder mit ihnen misohbar ist und im Dispersionsmittel praktisch unlöslich ist (Inertmittel), dadurch gekennzeichnet, daß A') 1 bis 99 Gew.-% Methacrylsäure-glycerincarbonat-ester, Acrylsäure-glycerincarbonat-ester, Glycerincarbonat-vinylether und/oder Glycerincarbonat-allylether und B') 99 bis 1 Gew.-% N,N'-Divinylethylenharnstoff und/oder N,N'-Divinylpropylenharnstoff, wobei die Summe der Monomeren stets 100 Gew.-% ist, in Gegenwart von 50 bis 300 Gew.-% Inertmittel, bezogen auf die Summe der Monomeren, copolymerisiert werden.

2. Verfahren nach Anspruch 1, dadurch gekennzeichnet, daß Methacrylsäure-glycerincarbonatester eingesetzt wird.

3. Verfahren nach Anspruch 1 oder 2, dadurch gekennzeichnet, daß zusätzlich als Komponente C') 0,1 bis 10 Gew.-%, bezogen auf das Monomerengemisch, Vinylacetat, Methylmethacrylat, Butylacrylat und/oder Styrol copolymerisiert werden.

4. Verfahren nach einem oder mehreren der Ansprüche 1 bis 3, dadurch gekennzeichnet, daß die Copolymerisation als Suspensionspolymerisation in Wasser als Suspensionsmittel bei einer Temperatur von 20 bis 120 °C durchgeführt wird.

5. Verwendung der Polymerisate nach Anspruch 1 als Trägermaterialien für die Immobilisierung biologisch aktiver Substanzen.

6. Verwendung nach Anspruch 5, dadurch gekennzeichnet, daß die biologisch aktiven Substanzen Enzyme sind.

**Revendications pour l'Etat contractant: AT**

1. Procédé pour la préparation du polymère ci-dessus, par copolymérisation des monomères dans un dispersant liquide qui, dans les conditions de la polymérisation, ne dissout pas les monomères et le polymère, en présence d'un amorceur radicalaire et d'autres adjuvants, de même que d'une substance qui est bien soluble dans les monomères ou leur est miscible, et n'est pratiquement pas soluble dans le dispersant (substance inerte), caractérisé en ce qu'on copolymérise A') 1 à 99% en poids, par rapport au mélange de monomères, d'ester méthacrylique et carbonique du glycérol, d'ester acrylique et carbonique du glycérol, d'éther vinylique-ester carbonique du glycérol et/ou d'éther allylique-ester carbonique du glycérol et B') de 99 à 1% en poids, par rapport au mélange de monomères, de N, N'-divinyléthylène-urée et/ou de N,N'-divinylpropylène-urée, la somme des monomères étant toujours de 100% en poids, en présence de 50 à 300% en poids, par rapport à la somme des monomères, de la substance inerte.

2. Procédé selon la revendication 1, caractérisé en ce qu'on utilise l'ester méthacrylique et carbonique du glycérol.

3. Procédé selon les revendications 1 ou 2, caractérisé en ce qu'on polymérise en outre en tant que composé C') 0,1 à 10% en poids, par rapport au mélange de monomères, d'acétate de vinyle, de méthacrylate de méthyle, d'acrylate de butyle et/ou de styrène.

4. Procédé selon l'une ou plusieurs des revendications 1 à 3, caractérisé en ce que la copolymérisation est mise en œuvre sous forme d'une polymérisation en suspension dans de l'eau servant d'agent de suspension, à une température de 20 à 120°C.

5. Utilisation des polymères selon la revendication 1 sous forme de supports pour immobiliser des substances biologiquement actives.

6. Utilisation selon la revendication 5, caractérisée en ce que les substances biologiquement actives sont des enzymes.

**Revendications pour les Etats contractants: BE, CH, DE, FR, GB, IT, LI, NL, SE**

1. Polymère réticulé, comprenant essentiellement A) de 1 à 99% en poids de motifs dérivant de l'ester méthacrylique et carbonique du glycérol, de l'ester acrylique et carbonique du glycérol, de l'éther vinylique-ester carbonique du glycérol et/ou de l'éther allylique-ester carboxylique du glycérol, B) de 99 à 1% en poids de motifs dérivant de la N,N'-divinyléthylène-urée et/ou de la N,N'-divinylpropylène-urée, la somme des motifs étant toujours de 100% en poids, les particules de polymères se présentant sous une forme essentiellement sphérique, ayant une granulométrie moyenne de 10 à 600 µm et un diamètre moyen des pores de 5 à 1000 nm.

2. Polymère selon la revendication 1, caractérisé en ce qu'on utilise l'ester méthacrylique et carbonique du glycérol.

3. Polymère selon la revendication 1 ou 2, caractérisé en ce qu'il contient en outre C) de 0,1 à 10% en poids, par rapport au polymère total, de motifs dérivant de l'acétate de vinyle, du méthacrylate de méthyle, de l'acrylate de butyle et/ou du styrène.

4. Procédé pour la préparation du polymère ci-dessus, par copolymérisation des monomères dans un dispersant liquide qui, dans les conditions de la polymérisation, ne dissout pas les monomères et le polymère, en présence d'un amorceur radicalaire et d'autres adjuvants, de même que d'une substance qui est bien soluble dans les monomères ou leur est miscible, et n'est pratiquement pas soluble dans le dispersant (substance inerte), caractérisé en ce qu'on copolymérise A') 1 à 99% en poids, par rapport au mélange de monomères, d'ester méthacrylique et carbonique du glycérol, d'ester acrylique et carbonique du glycérol, d'éther vinylique-ester carbonique du glycérol et/ou d'éther allylique-ester carbonique du glycérol et B') de 99 à 1% en poids, par rapport au mélange de monomères, de N,N'-divinyléthylène-urée et/ou de N,N'-divinylpropylène-urée, la somme des monomères étant toujours de 100% en poids, en présence de 50 à 300% en poids, par rapport à la somme des monomères, de la substance inerte.

5. Procédé selon la revendication 4, caractérisé en ce qu'on polymérise en outre en tant que composé C') 0,1 à 10% en poids, par rapport au mélange de monomères, d'acétate de vinyle, de méthacrylate de méthyle, d'acrylate de butyle et/ou de styrène.

6. Procédé selon l'une ou plusieurs des revendications 4 et 5, caractérisé en ce que la copolymérisation est mise en œuvre sous forme d'une polymérisation en suspension dans de l'eau servant d'agent de suspension, à une température de 20 à 120°C.

7. Utilisation des polymères selon la revendication 1 sous forme de supports pour immobiliser des substances biologiquement actives.

8. Utilisation selon la revendication 7, caractérisée en ce que les substances biologiquement actives sont des enzymes.

**Claims for the Contracting State AT**

1. A process for the preparation of a crosslinked polymer by copolymerization of monomers in a liquid dispersant which, under the polymerization conditions, does not dissolve the monomers and the polymer, in the presence of a free radical initiator and other auxiliaries, and of a substance which dissolves readily in, or is miscible with, the monomers and is virtually insoluble in the dispersant (inert agent), which comprises copolymerization of A') 1 to 99% by weight, of glycerol carbonate methacrylate, glycerol carbonate acrylate, glycerol carbonate vinyl ether and/or glycerol carbonate allyl ether and B') 99 to 1% by weight of N,N'-divinylethyleneurea and/or N,N'-divinylpropyleneurea, the total weight of the monomers always being 100% by weight, in the presence of 50 to 300% by weight, based on the total of the monomers, of inert agent.

2. The process as claimed in claim 1, wherein glycerol carbonate methacrylate is used.

3. The process as claimed in claim 1 or 2, wherein additionally as component C') 0.1 to 10% by weight, based on the monomer mixture, of vinyl acetate, methyl methacrylate, butyl acrylate and/or styrene is copolymerized.

4. The process as claimed in one or more of claims 1 to 3, wherein the copolymerization is carried out as suspension polymerization in water as suspending agent at a temperature of 20 to 120°C.

5. The use of the polymers as claimed in claim 1 as carrier materials for the immobilization of biologically active substances.

6. The use as claimed in claim 5, wherein the biologically active substances are enzymes.

**Claims for the Contracting States BE, CH, DE, FR, GB, IT, LI, NL, SE**

1. A crosslinked polymer substantially composed of A) 1 to 99% by weight of units derived from glycerol carbonate methacrylate, glycerol carbonate acrylate, glycerol carbonate vinyl ether and/or glycerol carbonate allyl ether, and B) 99 to 1% by weight of units derived from N,N'-divinylethyleneurea and/or N,N'-divinylpropyleneurea, with the total of the units always being 100% by weight, and with the polymer particles having substantially a spherical shape, a mean particle size of 10 to 600 μm and a mean pore diameter of 5 to 1000 nm.

2. A polymer as claimed in claim 1, wherein glycerol carbonate methacrylate is used.

3. A polymer as claimed in claim 1 or 2, which additionally contains C) 0.1 to 10% by weight, based on the total polymer, of units which are derived from vinyl acetate, methyl methacrylate, butyl acrylate and/or styrene.

4. A process for the preparation of a crosslinked polymer by copolymerization of monomers in a liquid dispersant which, under the polymerization conditions, does not dissolve the monomers and the polymer, in the presence of a free radical initiator and other auxiliaries, and of a substance which dissolves readily in, or is miscible with, the monomers and is virtually insoluble in the dispersant (inert agent), which comprises copolymerization of A') 1 to 99% by weight, of glycerol carbonate methacrylate, glycerol carbonate acrylate, glycerol carbonate vinyl ether and/or glycerol carbonate allyl ether and B') 99 to 1% by weight of N,N'-divinylethyleneurea and/or N,N'-divinylpropyleneurea, the total weight of the monomers always being 100% by weight, in the presence of 50 to 300% by weight, based on the total of the monomers, of inert agent.

5. The process as claimed in claim 4, wherein additionally as component C') 0.1 to 10% by weight, based on the monomer mixture, of vinyl acetate, methyl methacrylate, butyl acrylate and/or styrene is copolymerized.

6. The process as claimed in one or more of claims 4 and 5, wherein the copolymerization is carried out as suspension polymerization in water as suspending agent at a temperature of 20 to 120°C.

7. The use of the polymers as claimed in claim 1 as carrier materials for the immobilization of biologically active substances.

8. The use as claimed in claim 7, wherein the biologically active substances are enzymes.